# EUROPÄISCHE PATENTANMELDUNG

(11) **EP 0 543 091 A2**
(43) Veröffentlichungstag der Anmeldung: **26.05.1993**
(21) Anmeldenummer: 92113938.2
(22) Anmeldetag: 15.08.1992
(51) Int. Cl.: C12P 21/08, G01N 33/577, C12N 5/18

(54) **Monoklonaler Antikörper, der mit Pseudomonas aeruginosa reagiert**

(30) Priorität: 28.08.1991 DE 4128454
(71) Anmelder: RIEDEL-DE HAEN AKTIENGESELLSCHAFT, D-30926 Seelze (DE)
(72) Erfinder: Bitter-Suermann, Dieter, Prof. Dr., W-3000 Hannover 71 (DE)
(74) Vertreter: Muley, Ralf, Dr.

(57) **Zusammenfassung**

Die vorliegende Erfindung betrifft einen monoklonalen Antikörper, dadurch gekennzeichnet, daß er mit den Stämmen von Pseudomonas aeruginosa reagiert und nicht reagiert mit anderen Stämmen von Pseudomonas sowie anderen gram-negativen oder gram-positiven Bakterien.

## Beschreibung

Die vorliegende Erfindung betrifft einen monoklonalen Antikörper, der mit Pseudomonas aeruginosa reagiert, seine Verwendung zum Nachweis von Pseudomonas aeruginosa, ein ihn enthaltendes Testkit, sowie das ihn produzierende Hybridom.

Pseudomonas aeruginosa ist einer der häufigsten Erreger bei Infektionen geschwächter Patienten, wie beispielsweise Verbrennungspatienten, Leukämiepatienten oder Patienten mit cystischer Fibrose. Ein geeignetes, universell einsetzbares und einfach handhabbares Mittel zu dessen sicherem Nachweis ist bisher noch nicht bekannt.

Aufgabe der vorliegenden Erfindung ist es deshalb, ein solches Mittel bereitzustellen.

Es wurde nun gefunden, daß diese Aufgabe gelöst wird durch einen monoklonalen Antikörper, dadurch gekennzeichnet, daß er mit den Stämmen von Pseudomonas aeruginosa reagiert und nicht reagiert mit anderen Stämmen von Pseudomonas sowie anderen gram-negativen oder gram-positiven Bakterien.

Die erfindungsgemäßen monoklonalen Antikörper gehören bevorzugt den Klassen IgM, IgG und IgA an. Besonders bevorzugt gehören sie der Klasse IgG₁ an.

Besonders bevorzugte erfindungsgemäße monoklonale Antikörper sind solche, die ein spezies-spezifisches Epitop auf einem 60 KDa Heat-Shock-Protein, auch "Common-Antigen" genannt, das aus Pseudomonas aeruginosa isoliert werden kann, erkennen.

Ein spezies-spezifisches Epitop des genannten Heat-Shock-Proteins hat die Sequenz Gln-Ala-Asp-Ile-Glu-Ala-Arg-Val-Leu-Gln (SEQ ID NO: 1).

Ganz besonders bevorzugte erfindungsgemäße monoklonale Antikörper sind solche, die die genannte Sequenz erkennen. Ein Beispiel für einen solchen ganz besonders bevorzugten monoklonalen Antikörper ist der, der von dem unter der Hinterlegungsnummer 91 07 24 01 am 24.07.1991 bei der European Collection of Animal Cell Cultures in Porton Down, Salisbury, Wiltshire, SP4 0JG, United Kingdom hinterlegten Hybridom produziert wird.

Der von dem genannten hinterlegten Hybridom produzierte monoklonale Antikörper erkennt alle 17 in der "American Type Culture Collection" (ATCC) erfaßten Serumgruppen von Pseudomonas aeruginosa, aber keines der im folgenden genannten anderen Pseudomonaden bzw. andere gram-negative oder gram-positive Bakterien:
Acinetobacter
Bacteroides fragilis
Bordetella pertussis
Campylobacter jejuni
Citrobacter freundii
Enterobacter
Escherichia coli
Haemophilus influenzae Typ B
Klebsiella
Legionella pneumophila Serogruppe 1
Proteus vulgaris
Pseudomonas cepacia
Pseudomonas fluorescens
Pseudomonas maltophilia
Pseudomonas pickettii
Pseudomonas stutzeri
Salmonella typhimurium
Serratia liquefaciens
Shigella flexneri
Yersinia enterocolitica
Staphylococcus aureus
Streptokokken Typ B
Die genannten Bakterien stellen lediglich eine repräsentative Auswahl dar, die getestet wurde. Auch bei hier nicht genannten Bakterien wird keine Reaktivität mit dem genannten monoklonalen Antikörper erwartet.

Gegenstand der vorliegenden Erfindung sind auch Hybridome, die einen erfindungsgemäßen monoklonalen Antikörper produzieren.

Ganz besonders bevorzugt ist das unter der Hinterlegungs-Nummer 91 07 24 01 am 24.07.1991 bei der European Collection of Animal Cell Cultures in Porton Down, Salisbury, Wiltshire, SP4 0JG, United Kingdom hinterlegte Hybridom.

Die Herstellung monoklonale Antikörper produzierender Hybridome ist an sich bekannt und geht auf die Arbeiten von Köhler und Milstein (Nature 256, 495 - 97, 1975) zurück.

Im Rahmen der vorliegenden Erfindung werden dabei insbesondere die folgenden Verfahrensschritte ausgeführt:
a) Immunisieren einer Maus mit einem Lebendstamm von Pseudomonas aeruginosa durch intraperitoneale Injektion.
b) Entnahme der Milzzellen nach erfolgter Immunisierung.
c) Fusion der Milzzellen mit Maus-Myelom-Zellen P3-X63-Ag8.653.
d) Selektion der Hybridoma-Zellen.
e) Clonen und Kultivieren der Hybridome.
f) Testen und Selektieren der Hybridome.
g) Kultivieren des selektierten Hybridoms.

Die Gewinnung des monoklonalen Antikörpers geschieht dann bevorzugt durch Expansion des entsprechenden Hybridoms in der Ascites-Maus oder durch Propagation des Hybridoms in Zellkultur und Reinigung aus der Ascites-Flüssigkeit bzw. dem Zellkultur-Überstand.

Die erfindungsgemäßen monoklonalen Antikörper eignen sich in hervorragender Weise zum immunologischen Nachweis von Pseudomonas aeruginosa.

Bevorzugte Methoden zu diesem Nachweis sind Radioimmunoassay (RIA), enzymgekoppelter Assay (ELISA), insbesondere Sandwich-ELISA und Chemolumineszenzassay. Die genannten Methoden sind dem Fachmann bekannt und in der Fachliteratur beschrieben.

Besonders vorteilhaft zum immunologischen Nachweis von Pseudomonas aeruginosa ist ein gebrauchsfertiger Testkit, der neben den üblicherweise verwendeten Reagentien, Trägermaterialien und sonstigen Zusätzen als immunologisches Reagenz mindestens einen der erfindungsgemäßen monoklonalen Antikörper enthält.

In den folgenden Beispielen wurden die nachstehenden Reagenzien bzw. Methoden verwendet:
- Heart-Brain-Bouillon
   37 g Brain-Heart-Infusion (Fa. Oxoid) auf 1 Liter Wasser
- PBS = Phosphat buffered Saline
   10 mM Natriumphosphat-Puffer, pH 7,6; 0,9 % NaCl
- DMEM = Dulbecco's Modified Eagle Medium
   (Flow Lab, Meckenheim)
- Normalmedium
   DMEM mit 2 mM Glutamin (Gibco, Paisley Scotland)
   50 I.E./ml Penicillin (Gibco, Paisley Scotland)
   50 µg/ml Streptomycin (Gibco, Paisley Scotland)
- HAT-Selektionsmedium
   Normalmedium mit
   100 µM Hypoxanthin
   16 µM Thymidin
   0,4 µM Aminopterin
   50 U/ml Interleukin 6 (Boehringer Mannheim)
- PDL-beschichtete Mikrotiterplatten
   Polystyrol-Mikrotiterplatten (Fa. Greiner, Nürtingen)
   mit 50 µl PDL-Lösung (25 µg/ml Poly-(DL) -Lysin (Sigma, München) in Puffer A) pro Kavität beschichten,
   1 h bei Raumtemperatur inkubieren,
   3 x mit Puffer A waschen
- Glutaraldehydlösung
   0,05 % Glutardialdehyd (Fa. Merck, Darmstadt) in Puffer A
- Puffer A
   10 mM Kaliumphosphatpuffer, pH 6,0
- Puffer A-BSA
   Puffer A; 1 % (Gewichstprozent) Rinderserumalbumin (Boehringer Mannheim)
- Puffer B
   10 mM Kaliumphosphatpuffer, pH 6,0
- Peroxidase-Substrat-Lösung
   2,7 % 2,2'-Azinodi(3-ethylbenzthiazolinsulfonic acid) (Fa. Sigma, München); 0,0025 % H₂O₂ in Puffer B
- Columbia-Blut-Agar-Platten mit 5 % Schafsblut (Fa. Becton-Dickinson)
- SDS-PAGE Proben-Puffer (SDS-PAGE = Sodiumdodecylsulfatpolyacrylamidgel-electrophorese)
   62,5 mM Tris-(hydroxymethyl)-aminomethan-HCl, pH 6,8
   5 % 2-Mercaptoethanol
   2 % SDS (Natriumdodecylsulfat, Fa. Merck)
   12,5 % Glycerin
- Kolonie-Hybridisierung
   Nitrocellulosefilter auf Agarplatte auflegen und abziehen,
   10 min bei 37^{o}C trocknen,
   Filter befeuchten mit:
   0,5 M NaOH, 2 min Raumtemperatur (RT)
   1,5 M NaCl, 0,5 M Tris-(hydroxymethyl)-aminomethan-HCL,
   pH 7,5; 2 x 2 min RT
   180 mM NaCl, 5 mM Na₂HPO₄, 5 mM NaH₂PO₄, 1 mM EDTA,
   2 x 2 min RT
   70 % Ethanol
   Filter 10 min bei 37^{o}C trocknen,
   Filter wie Immunoblot mit Antikörper behandeln und entwickeln.

### Beispiel 1

### Herstellung eines Hybridoms

### a) Immunisierung

Der Stamm Pseudomonas aeruginosa DSM 1707 wird auf Heart-Brain-Bouillon angezüchtet. Die Bakterien werden geerntet, zweimal gewaschen und suspendiert in PBS. Zur Immunisierung werden 6 - 8 Wochen alten BALB/C-Mäusen im Laufe mehrerer Wochen Suspensionen von DSM 1707 wie angegeben intraperitoneal injiziert:

| Woche | DSM 1707 | Applikation (i. p.) |
|---|---|---|
| 1 | 1 x 10⁶ Lebendkeime | komplettes Freund Adjuvans |
| 2 | 5 x 10⁶ Lebendkeime | inkomplettes Freund Adjuvans |
| 3 | 25 x 10⁶ Lebendkeime | 0,9 % NaCl |
| 4 | 125 x 10⁶ Lebendkeime | 0,9 % NaCl |
| 5 | 625 x 10⁶ Lebendkeime | 0,9 % NaCl |
| 6 | 3,13 x 10⁹ Lebendkeime | 0,9 % NaCl |

### b) Somatische Zellfusion zur Herstellung des Hybridoms

Die Milz einer immunisierten Maus wird 3 - 4 Tage nach der letzten Booster-Injektion unter sterilen Bedingungen entnommen. Durch vorsichtiges Zerreiben auf einem Edelstahl-Sieb (Porenweite 100 µm) werden die Milzzellen aus dem Gewebeverband isoliert und in 1 ml DMEM aufgenommen. Die Zellsuspension wird anschließend einmal mit 5 ml DMEM gewaschen (Zentrifugation bei 1200 rpm, 7 Minuten) und in 5 ml DMEM suspendiert. Myelomzellen (Zellinie P3-X63-Ag8.653) werden in Normal Medium mit einem Anteil von 1 % FCS gezüchtet, in DMEM Medium dreimal gewaschen und suspendiert. 5 x 10⁷ Milzzellen werden mit 5 x 10⁷ Myelomzellen in DMEM Medium gemischt und bei 800 rpm 10 Minuten abzentrifugiert. Der Überstand wird abgesaugt und das Sediment in einem Wasserbad (37^{o}C) langsam mit 800 µl einer 37^{o}C warmen, sterilen 100 %igen Polyethylenglycollösung (PEG 1500, Boehringer Mannheim) versetzt, um die Fusion der Zellpopulation zu starten. Nach 1 Minute wird das Polyethylenglykol durch Zugabe von DMEM ausverdünnt und das Sediment nach Zentrifugation (1200 rpm, 5 Minuten) in HAT Selektiv Medium mit einer Zelldichte von 1 x 10⁶ Zellen pro ml aufgenommen.

In diesem Selektions Medium überleben ausschließlich fusionierte Zellen, nicht jedoch die ebenfalls noch in der Suspension befindlichen unfusionierten Milz- bzw. Maus-Myelom-Zellen P3-X63-AG8.653. Anschließend wird die Zellsuspension auf 96 Loch Gewebekulturplatten (Fa. Costar, Fernwald) zu je 200µl pro Loch verteilt. Die Hybridoma Kulturen werden im Brutschrank bei 37^{o}C und 7,5 % CO₂ inkubiert.

### c) Hybridoma Screening (ELISA)

Zur Testung der Kulturüberstände der Hybridoma-Kulturen wird der im folgenden beschriebene ELISA eingesetzt. DSM 1707 werden auf Blutplatten gezüchtet, in physiologischer Kochsalzlösung gewaschen und auf 2 x 10⁸ Lebendkeime pro ml eingestellt. Diese Suspension wird zu 20 µl pro Kavität auf PDL-beschichtete Mikrotiterplatten pipettiert. Nach Inkubation bei Raumtemperatur für 1 Stunde werden 10 µl Glutaraldehydlösung zupipettiert. Nach 10 Minuten werden die Platten mit Puffer A zweimal gewaschen; noch freie Bindungsstellen werden mit Puffer A-BSA während 30 Minuten bei Raumtemperatur abgesättigt. Die gewaschenen Platten werden mit 20 µl Hybridoma-Kulturüberstand pro Kavität 2 Stunden bei Raumtemperatur inkubiert. Die Detektion gebundener Antikörper erfolgt mittels 10 µl Peroxidase-markierten Ziege-Antimaus-Immunoglobulin (Boehringer Mannheim) während 30 Minuten bei Raumtemperatur. Nach dem Waschen der Mikrotiterplatte (zweimal Puffer A, einmal Puffer B) wird die Enzymaktivität durch Zugabe von 20 µl Substratlösung pro Kavität, Inkubation bei Raumtemperatur und Bestimmung der Absorptionsänderung bei 405 nm ermittelt. Hybridomazellen, die an DSM 1707 bindende Antikörper sezernieren, werden nach dem Prinzip des "Limiting Dilution" kloniert, um sicherzustellen, daß die Zellen nur von einem Hybridzellklon stammen, d. h. monoklonal sind.

### Beispiel 2

### Gewinnung und Charakterisierung des von dem hinterlegten Hybridom produzierten Antikörpers

### a) Expansion in der Ascites-Maus (in vivo)

Mäuse vom Stamm BALB/C erhalten zur Konditionierung des Peritoneums 0,5 ml Pristan (2,6,10,14-Tetramethylpentadecan; Aldrich Chemie, Steinheim) intraperitoneal. 3 bis 10 Tage später werden den so vorbehandelten Tieren Suspensionen von 5 x 10⁵ -1 x 10⁶ Hybridomazellen gemäß Beispiel 1c) in PBS intraperitoneal injiziert. Nach 10 bis 20 Tagen wird mit einer Kanüle das Peritoneum angestochen und die Ascitesflüssigkeit gesammelt. Die zellulären Bestandteile wurden durch Zentrifugation abgetrennt (10.000 rpm, 5 min), der Überstand, der auch die monoklonalen Antikörper enthält, abgenommen und bei -70^{o}C aufbewahrt.

### b) Kreuzreaktivität

Zunächst wird die Subklasse des nach Beispiel 2a) hergestellten monoklonalen Antikörpers bestimmt. Dafür werden im ELISA (siehe Beispiel 1c)) anstelle von Peroxidase-markiertem Anti-Maus-Immunglobulin Subklassenspezifische Peroxidase-Konjugate (Boehringer Mannheim) eingesetzt. Der in der Ascitesflüssigkeit enthaltene monoklonale Antikörper wird entsprechend seiner Subklasse durch Affinitätsreinigung an Protein A-bzw. Protein G-Sepharose (Pharmacia, Freiburg) gewonnen.

Zur Bestimmung der Antikörper-Spezifität werden intakte Bakterien im ELISA eingesetzt. Durch SDS-PAGE und Immunoblot läßt sich das Molekulargewicht des Antigens bestimmen bzw. eingrenzen. Hierzu werden auf Columbia-Blut-Agar-Platten angezüchtete Bakterien in PBS zweimal gewaschen und abzentrifugiert. 2 x 10⁹ Bakterien pro ml werden in 1 ml SDS-PAGE-Proben-Puffer suspendiert und 4 x 15 sec lang mit Ultraschall (Branson Sonicator, Mikrospitze) aufgeschlossen. Nach 10 min bei 100^{o}C werden 20 µl Lösung durch SDS-PAGE (12 % Polyacrylamid) aufgetrennt und nach Towbin H., Staehelin T. und Gordon J. (1979) Proc. Natl. Acad. Sci. USA 76,4350-4354 auf Nitrocellulose-Membranen übertragen und mittels Antikörper detektiert.

Der monoklonalen Antikörper zeigt zwischen ELISA und Immunoblot keine Unterschiede in der Immunreaktivität. Seine Immunreaktivität gegenüber exemplarisch ausgewählten Bakterienstämmen ist bereits in der Beschreibung genannt. Er erfüllt die eingangs genannte Aufgabe, ein Nachweismittel für Pseudomonas aeruginosa zur Verfügung zu stellen, indem er die verschiedenen Serumgruppen von Pseudomonas aeruginosa, sowie willkürlich ausgewählte Patientenisolate von Pseudomonas aeruginosa (20 schleimbildende Isolate von CF-Patienten und 20 nicht-schleimbildende Wund-Isolate sowie 5 Laborstämme, abgeleitet aus Isolaten von CF-Patienten) erkennt. In allen diesen Fällen erkennt der Antikörper ein 60 kDa-Protein. Repräsentative gram-negative und gram-positive Bakterienstämme sowie andere Pseudomonaden erkennt er nicht.

### c) Epitop-Charakterisierung

Zur Epitop-Charakterisierung eignen sich neben proteinchemischen Methoden gentechnologische Methoden und DNA-Sequenzanalyse. Vorraussetzung ist die Identifizierung des Strukturgens bzw. eines Teils davon. Ein monoklonaler Antikörper, der ein Protein-Epitop auch unter denaturierenden Bedingungen (Immunoblot) erkennt, ermöglicht die Isolierung des zugehörigen Strukturgens aus einer Genbank über Kolonie-Hybridisierung. Nach Clonierung läßt sich das erkannte Epitop durch partiellen Abbau mit Exonuclease (Nested-Deletion-Kit; Pharmacia, Freiburg) eingrenzen; die Bestimmung der Peptidsequenz ist über eine DNA-Sequenzanalyse leicht möglich.

Eine genauere Eingrenzung des Epitops ist über synthetische Oligonukleotide (Gene Assembler Plus Oligonucleotide Synthesizer; Pharmacia, Freiburg) und ihre Expression als Fusionsprotein (Glutathion-S-Transferase) mittels Expressions-Vektor (z. B. pGEX-3X; Pharmacia, Freiburg) möglich. Gemäß o. g. Strategie läßt sich beispielhaft das erkannte Epitop des in 2a) und 2b) beschriebenen monoklonalen Antikörpers eingrenzen. Er erkennt ein Deca-Peptid -Gln-Ala-Asp-Ile-Glu-Ala-Arg-Val-Leu-Gln - (SEQ ID NO: 1) entsprechend den Aminosäuren Nr. 339 bis 348 des GroEL-Proteins von Pseudomonas aeruginosa.

### Beispiel 3

### Nachweis von Pseudomonas aeruginosa in Wasserproben

100 ml einer steril entnommenen Wasserprobe werden membranfiltriert (0,2 µm, ME 24/21, Schleicher & Schüll) und in 10 ml DEV-Lactose-Pepton-Bouillon bei 36 ± 1^{o}C inkubiert. Nach 20 Stunden Inkubationszeit wird 1 ml Aliquot entnommen und 4 x 15 sec beschallt (Branson Sonifier, Mikrospitze). Der ELISA zum Nachweis von Pseudomonas aeruginosa wird bei Raumtemperatur durchgeführt. Mikrotiterplatten (Polystyrol, Greiner) werden mit 125 µl Antikörperlösung pro Kavität (20 µg Antikörper-Protein pro ml PBS) beschichtet (120 min RT oder 4^{o}C über Nacht) und zweimal mit Puffer A gewaschen. Nach dem Absättigen (250 µl Puffer A-BSA, 30 min RT) und zweimaligem Waschen mit Puffer A werden 100 µl der vorbereiteten Probe pro Kavität zugegeben. Nach 2 Stunden bei Raumtemperatur und dreimaligem Waschen mit Puffer A werden 100 µl einer Lösung von enzymmarkiertem monoklonalen Antikörper gemäß Beispiel 2 pro Kavität einpipettiert. Nach einer Stunde bei Raumtemperatur und dreimaligem Waschen mit Puffer A werden 125 µl einer geeigneten Enzymsubstratlösung aufpipettiert und die Platte nach einer angemessenen Inkubationszeit optisch oder fluorimetrisch ausgemessen.

Sequenzprotokoll

SEQ ID NO: 1
Aminosäuresequenz aus 10 Aminosäuren.
Lineares Peptid entsprechend den Aminosäuren Nr. 339 bis
348 des GroEL-Proteins von Pseudomonas aeruginosa.

Gln Ala Asp Ile Gln Ala Arg Val Leu Gln
1               5                   10

## Patentansprüche

1. Monoklonaler Antikörper, dadurch gekennzeichnet, daß er mit den Stämmen von Pseudomonas aeruginosa reagiert und nicht reagiert mit anderen Stämmen von Pseudomonas sowie anderen gram-negativen oder gram-positiven Bakterien.

2. Monoklonaler Antikörper gemäß Anspruch 1, dadurch gekennzeichnet, daß er aus der Klasse IgG₁ ist.

3. Monoklonaler Antikörper gemäß Anspruch 1 und/oder 2, dadurch gekennzeichnet, daß er ein spezies-spezifisches Epitop auf einem 60 kDa Heat-Shock-Protein von Pseudomonas aeruginosa erkennt.

4. Monoklonaler Antikörper gemäß einem oder mehreren der Ansprüche 1 bis 3, dadurch gekennzeichnet, daß er die Proteinsequenz Gln-Ala-Asp-Ile-Glu-Ala-Arg-Val-Leu-Gln (SEQ ID NO: 1) erkennt.

5. Monoklonaler Antikörper gemäß einem oder mehreren der Ansprüche 1 bis 4, dadurch gekennzeichnet, daß er produziert worden ist von dem Hybridom, das unter der Hinterlegungsnummer 91 07 24 01 am 24.07.1991 bei der European Collection of Animal Cell Cultures in Porton Down, Salisbury, Wiltshire, SP4 0JG, United Kingdom hinterlegt wurde.

6. Hybridom, dadurch gekennzeichnet, daß es einen monoklonalen Antikörper gemäß einem der Ansprüche 1 bis 5 produziert.

7. Hybridom hinterlegt unter der Hinterlegungsnummer 91 07 24 01 am 24.07.1991 bei der European Collection of Animal Cell Cultures in Porton Down, Salisbury, Wiltshire, SP4 0JG, United Kingdom.

8. Verfahren zur Herstellung eines Hybridoms gemäß Anspruch 6 und/oder 7, dadurch gekennzeichnet, daß die folgenden Verfahrensschritte ausgeführt werden:
a) Immunisieren einer Maus mit einem Lebendstamm von Pseudomonas aeruginosa durch intraperitoneale Injektion.
b) Entnahme der Milzzellen nach erfolgter Immunisierung.
c) Fusion der Milzzellen mit Maus-Myelom-Zellen P3-X63-Ag8.653.
d) Selektion der Hybridoma-Zellen.
e) Clonen und Kultivieren der Hybridome.
f) Testen und Selektieren der Hybridome.
g) Kultivieren des selektierten Hybridoms.

9. Verwendung eines monoklonalen Antikörpers gemäß einem der Ansprüche 1 bis 5 zum immunologischen Nachweis von Pseudomonas aeruginosa.

10. Testkit zum immunologischen Nachweis von Pseudomonas aeruginosa, dadurch gekennzeichnet, daß er neben den üblicherweise verwendeten Reagentien, Trägermaterialien und sonstigen Zusätzen als immunologisches Reagenz mindestens einen monoklonalen Antikörper gemäß einem der Ansprüche 1 bis 5 enthält.
